# EUROPEAN PATENT APPLICATION

(11) **EP 2 508 618 A1**
(43) Date of publication of application: **10.10.2012**
(21) Application number: 11160949.1
(22) Date of filing: 04.04.2011
(51) Int. Cl.: C12Q 1/68

(54) **Methods and means for determining a functional polymorphism in ficolin-2 for predicting the outcome of kidney transplantation**

(71) Applicant: Innogenetics N.V., 9052 Ghent (BE)
(72) Inventor: Eikmans, Michael, 2343 BN, Oegstgeest (NL); De Canck, Ilse, 2610, Wilrijk (BE); Claas, Frans, 2353 JH, Leiderdorp (NL)
(74) Representative: BiiP cvba

(57) **Abstract**

The present invention provides a method for prediction of renal transplantation outcome, for assessment of the risk of post-transplantation kidney rejection, and/or for selecting an appropriate treatment, therapeutic agent or therapeutic agent dosage regime for the treatment of post-transplantation kidney rejection, whereby the presence and/or absence of at least one nucleic acid polymorphism in the gene for ficolin-2 of the kidney is detected. Further provided are oligonucleotides and kits for use in such method.

SEQUENCE LISTING

<110>  INNOGENETICS nv
       Eikmans, Michael
       De Canck, Ilse
       Claas, Frans

<120>  METHODS AND MEANS FOR DETERMINING A FUNCTIONAL POLYMORPHISM IN
       FICOLIN-2 FOR PREDICTING THE OUTCOME OF KIDNEY TRANSPLANTATION

<130>  INNX-208

<160>  2

<170>  PatentIn version 3.3

<210>  1
<211>  1057
<212>  RNA
<213>  Homo sapiens


<220>
<221>  5'UTR
<222>  (1)..(10)

<220>
<221>  CDS
<222>  (11)..(952)

<220>
<221>  3'UTR
<222>  (953)..(1057)

<400>  1
accagaagag aug gag cug gac aga gcu gug ggg guc cug ggc gcu gcc        49
           Met Glu Leu Asp Arg Ala Val Gly Val Leu Gly Ala Ala
           1               5                   10

acc cug cug cuc ucu uuc cug ggc aug gcc ugg gcu cuc cag gcg gca        97
Thr Leu Leu Leu Ser Phe Leu Gly Met Ala Trp Ala Leu Gln Ala Ala
    15                  20                  25

gac acc ugu cca gag gug aag aug gug ggc cug gag ggc ucu gac aag       145
Asp Thr Cys Pro Glu Val Lys Met Val Gly Leu Glu Gly Ser Asp Lys
30                  35                  40                  45

cuc acc auu cuc cga ggc ugu ccg ggg cug ccu ggg gcc ccu ggg ccc       193
Leu Thr Ile Leu Arg Gly Cys Pro Gly Leu Pro Gly Ala Pro Gly Pro
                50                  55                  60

aag gga gag gca ggc acc aau gga aag aga gga gaa cgu ggc ccc ccu       241
Lys Gly Glu Ala Gly Thr Asn Gly Lys Arg Gly Glu Arg Gly Pro Pro
            65                  70                  75

gga ccu ccu ggg aag gca gga cca ccu ggg ccc aac gga gca ccu ggg       289
Gly Pro Pro Gly Lys Ala Gly Pro Pro Gly Pro Asn Gly Ala Pro Gly
        80                  85                  90

gag ccc cag ccg ugc cug aca ggc ccg cgu acc ugc aag gac cug cua       337
Glu Pro Gln Pro Cys Leu Thr Gly Pro Arg Thr Cys Lys Asp Leu Leu
    95                  100                 105

gac cga ggg cac uuc cug agc ggc ugg cac acc auc uac cug ccc gac       385
Asp Arg Gly His Phe Leu Ser Gly Trp His Thr Ile Tyr Leu Pro Asp
110                 115                 120                 125

ugc cgg ccc cug acu gug cuc ugu gac aug gac acg gac gga ggg ggc       433

Cys Arg Pro Leu Thr Val Leu Cys Asp Met Asp Thr Asp Gly Gly
                130                 135                 140

ugg acc guu uuc cag cgg agg gug gau ggc ucu gug gac uuc uac cgg 481
Trp Thr Val Phe Gln Arg Arg Val Asp Gly Ser Val Asp Phe Tyr Arg
                145                 150                 155

gac ugg gcc acg uac aag cag ggc uuc ggc agu cgg cug ggg gag uuc 529
Asp Trp Ala Thr Tyr Lys Gln Gly Phe Gly Ser Arg Leu Gly Glu Phe
            160                 165                 170

ugg cug ggg aau gac aac auc cac gcc cug acc gcc cag gga acc agc 577
Trp Leu Gly Asn Asp Asn Ile His Ala Leu Thr Ala Gln Gly Thr Ser
        175                 180                 185

gag cuc cgu gua gac cug gug gac uuu gag gac aac uac cag uuu gcu 625
Glu Leu Arg Val Asp Leu Val Asp Phe Glu Asp Asn Tyr Gln Phe Ala
190 195 200 205

aag uac aga uca uuc aag gug gcc gac gag gcg gag aag uac aau cug 673
Lys Tyr Arg Ser Phe Lys Val Ala Asp Glu Ala Glu Lys Tyr Asn Leu
210 215 220

guc cug ggg gcc uuc gug gag ggc agu gcg gga gau ucc cug acg uuc 721
Val Leu Gly Ala Phe Val Glu Gly Ser Ala Gly Asp Ser Leu Thr Phe
                225                 230                 235

cac aac aac cag ucc uuc ucc acc aaa gac cag gac aau gau cuu aac 769
His Asn Asn Gln Ser Phe Ser Thr Lys Asp Gln Asp Asn Asp Leu Asn
            240                 245                 250

acc gga aau ugu gcu gug aug uuu cag gga gcu ugg ugg uac aaa aac 817
Thr Gly Asn Cys Ala Val Met Phe Gln Gly Ala Trp Trp Tyr Lys Asn
        255                 260                 265

ugc cau gug uca aac cug aau ggu cgc uac cuc agg ggg acu cau ggc 865
Cys His Val Ser Asn Leu Asn Gly Arg Tyr Leu Arg Gly Thr His Gly
270 275 280 285

agc uuu gca aau ggc auc aac ugg aag ucg ggg aaa gga uac aau uau 913
Ser Phe Ala Asn Gly Ile Asn Trp Lys Ser Gly Lys Gly Tyr Asn Tyr
290 295 300

agc uac aag gug uca gag aug aag gug cga ccu gcc uag cccaggccgg 962
Ser Tyr Lys Val Ser Glu Met Lys Val Arg Pro Ala
                305                 310

ccucaggguc aggacgccuc cacacauagu ugguuggggg guaggguugg gagcuuggcc 1022

cuacgguuug uaaaagaaac acaugucgug auucu 1057

<210> 2
<211> 313
<212> PRT
<213> Homo sapiens

<400> 2

Met Glu Leu Asp Arg Ala Val Gly Val Leu Gly Ala Ala Thr Leu Leu
1               5                   10                  15

Leu Ser Phe Leu Gly Met Ala Trp Ala Leu Gln Ala Ala Asp Thr Cys
            20                  25                  30

Pro Glu Val Lys Met Val Gly Leu Glu Gly Ser Asp Lys Leu Thr Ile

        35                  40                  45

Leu Arg Gly Cys Pro Gly Leu Pro Gly Ala Pro Gly Pro Lys Gly Glu
    50                  55                  60

Ala Gly Thr Asn Gly Lys Arg Gly Glu Arg Gly Pro Pro Gly Pro Pro
65                  70                  75                  80

Gly Lys Ala Gly Pro Pro Gly Pro Asn Gly Ala Pro Gly Glu Pro Gln
                85                  90                  95

Pro Cys Leu Thr Gly Pro Arg Thr Cys Lys Asp Leu Leu Asp Arg Gly
            100                 105                 110

His Phe Leu Ser Gly Trp His Thr Ile Tyr Leu Pro Asp Cys Arg Pro
        115                 120                 125

Leu Thr Val Leu Cys Asp Met Asp Thr Asp Gly Gly Gly Trp Thr Val
    130                 135                 140

Phe Gln Arg Arg Val Asp Gly Ser Val Asp Phe Tyr Arg Asp Trp Ala
145                 150                 155                 160

Thr Tyr Lys Gln Gly Phe Gly Ser Arg Leu Gly Glu Phe Trp Leu Gly
                165                 170                 175

Asn Asp Asn Ile His Ala Leu Thr Ala Gln Gly Thr Ser Glu Leu Arg
            180                 185                 190

Val Asp Leu Val Asp Phe Glu Asp Asn Tyr Gln Phe Ala Lys Tyr Arg
        195                 200                 205

Ser Phe Lys Val Ala Asp Glu Ala Glu Lys Tyr Asn Leu Val Leu Gly
    210                 215                 220

Ala Phe Val Glu Gly Ser Ala Gly Asp Ser Leu Thr Phe His Asn Asn
225                 230                 235                 240

Gln Ser Phe Ser Thr Lys Asp Gln Asp Asn Asp Leu Asn Thr Gly Asn
                245                 250                 255

Cys Ala Val Met Phe Gln Gly Ala Trp Trp Tyr Lys Asn Cys His Val
            260                 265                 270

Ser Asn Leu Asn Gly Arg Tyr Leu Arg Gly Thr His Gly Ser Phe Ala
        275                 280                 285

Asn Gly Ile Asn Trp Lys Ser Gly Lys Gly Tyr Asn Tyr Ser Tyr Lys
    290                 295                 300

Val Ser Glu Met Lys Val Arg Pro Ala

305                 310

## Description

### FIELD OF THE INVENTION

The present invention is related to the field of organ transplantation. More in particular, the present invention is related to renal transplantation.

### BACKGROUND ART

The degree by which an immune response is induced in solid organ transplants varies between individuals. This variation is determined partly by DNA sequence differences in promoter and coding regions of immunologically relevant genes. To reduce the risk of rejection, the HLA type of the donor and the recipient should be as similar as possible. HLA matching alone is not enough, since even in perfectly matched donor recipient pairs rejection can occur. Other markers like innate immunity genes are possibly involved.

The innate immune system acts as a first line of host defense against infections, but it is also involved in clearance of potentially dangerous substances arising from cellular debris and damaged cells within the host itself. Invading pathogens give rise to pathogen-associated molecular patterns (PAMPs) that are detected by pattern recognition receptors (PRRs). In a similar manner, danger signals or damage-associated molecular pattern (DAMPs), released by cells in response to damage or stress, can be bound by PRRs. The PRRs are present in the cell membrane or may be present in soluble form in the circulation. The group of cell-bound receptors is formed by the Toll-like receptors (TLRs), which are prominently expressed by antigen presenting cells and natural killer cells. The group of soluble recognition molecules includes Clq, mannose-binding lectin (MBL), and the ficolins (FCN), which can all activate complement.

Current mechanistic paradigms focus on the role of adaptive immunity in directing organ rejection. However, within the context of kidney transplantation PRR signaling may be important in priming of the alloimmune response. Pre- and post-operative, antigen-independent insult to the donor organ, such as ischemia-reperfusion damage and oxidative stress, may lead to the release of intracellular contents from necrotic cells. Such contents include high mobility group box-1 (HMGB1), heparan sulfate, hyaluronan, fibrinogen, and heat-shock proteins, which may act as endogenous ligands activating PRRs (Scaffidi et al., 2002; Goldstein, 2007). Several PRRs including MBL, FCN2, FCN3, and C1q can engage apoptotic and necrotic cells (Kuraya et al., 2005; Jensen et al., 2007; Nauta et al., 2003; Ogden et al., 2001), and in this way may participate in removal of dying cells and maintenance of tissue homeostasis.

Upon ischemic injury of solid organ transplants PRRs potentially modulate the extent of inflammation (Kruger et al., 2009; Leemans et al., 2005). Donor kidneys with a TLR4 loss-of-function allele, conferring diminished affinity for HMGB1, contain lower expression of proinflammatory cytokines and exhibited a higher rate of immediate graft function (Kruger et al., 2009). PRRs play a role in rejection of organ transplants (Alegre et al., 2008; Goldstein, 2006; Bouwman et al., 2006; Obhrai and Goldstein, 2006). Single nucleotide polymorphisms (SNPs) in the TLR4 gene were found to be associated with a reduced incidence of acute rejection in lung transplants (Palmer et al., 2003) and in kidney transplants (Palmer et al., 2006; Ducloux et al., 2005). Superior graft survival in kidney and combined kidney-pancreas transplantation is associated with low MBL serum levels (Berger et al., 2007; Berger et al., 2005), which can result from a SNP in exon 1 of the MBL2 gene (Berger et al., 2005).

Still, the prediction of renal transplantation outcome or the assessment of the risk of post-transplantational kidney rejection needs to be improved, as an enhanced prediction or risk assessments will allow to better select the optimal post-transplantational treatment scheme.

### SUMMARY OF THE INVENTION

The present invention provides a method for prediction of renal transplantation outcome, for assessment of the risk of post-transplantation kidney rejection, and/or for selecting an appropriate treatment, therapeutic agent or therapeutic agent dosage regime for the treatment of post-transplantation kidney rejection.

The method of the invention can be carried out in combination with other methods for prediction of renal transplantation outcome, for assessment of the risk of post-transplantation kidney rejection, and/or for selecting an appropriate treatment, therapeutic agent or therapeutic agent dosage regime for the treatment of post-transplantation kidney rejection.

In a first embodiment, the method of the invention comprises detecting the presence and/or absence of at least one nucleic acid polymorphism in the gene for ficolin-2 of the kidney.

In a preferred embodiment, the method of the invention comprises detecting the presence and/or absence of at least one nucleic acid polymorphism in the gene for ficolin-2 of the kidney which results in altered binding capacity of the protein encoded by the gene for ficolin-2 to N-acetyl-D-glucosamine (GlcNAc).

In a more preferred embodiment, the method of the invention comprises detecting the presence and/or absence of at least one nucleic acid polymorphism in the gene for ficolin-2 of the kidney which results in substitution of alanine on position 258 of the protein encoded by the gene for ficolin-2 by another amino acid.

In an even more preferred embodiment, the method of the invention comprises detecting the presence and/or absence of at least one nucleic acid polymorphism in the gene for ficolin-2 of the kidney which results in substitution of alanine on position 258 of the protein encoded by the gene for ficolin-2 by serine.

In a preferred embodiment, the method of the invention comprises detecting the presence and/or absence of at least one SNP in the gene for ficolin-2 of the kidney.

In a more preferred embodiment, the method of the invention comprises detecting the presence and/or absence of at least SNP rs7851696 in the gene for ficolin-2 of the kidney.

In a preferred embodiment, the method of the invention comprises detecting the presence and/or absence of at least one nucleic acid polymorphism in the gene for ficolin-2 of the kidney, whereby
- the kidney being heterozygous for the presence of said nucleic acid polymorphism is indicative of improved prognosis of renal transplantation outcome, of decreased risk of post-transplantation kidney rejection, and/or of decreased need of immunosuppression therapy after transplantation, and
- the kidney being homozygous for the absence of said nucleic acid polymorphism is indicative of poorer prognosis of renal transplantation outcome, of increased risk of post-transplantation kidney rejection, and/or of increased need of immunosuppression therapy after transplantation.

More preferred methods for detecting the presence and/or absence of at least one nucleic acid polymorphism in the gene for ficolin-2 use at least one nucleic acid polymorphism-specific oligonucleotide, preferably at least a SNP rs7851696-specific oligonucleotide.

Also part of the invention are methods for prediction of renal transplantation outcome, for assessment of the risk of post-transplantation kidney rejection, and/or for selecting an appropriate treatment, therapeutic agent or therapeutic agent dosage regime for the treatment of post-transplantation kidney rejection, comprising determining the ligand-binding capacity, preferably GlcNAc-binding capacity, of ficolin-2 from the kidney. The method of the invention can be performed *in vivo* or *in vitro.*

Also part of the present invention are ficolin-2 polymorphism-specific oligonucleotides, preferably SNP rs7851696-specific oligonucleotides, for use in the methods of the invention. Oligonucleotides of the invention are preferably primers or probes. Oligonucleotides of the invention may be immobilized to a solid phase. Also part of the invention kits for use in the methods of the invention, comprising such ficolin-2 polymorphism-specific oligonucleotides. Preferably, the kits of the invention further comprise at least one oligonucleotide which is specific for the wild-type allele corresponding to said ficolin-2 polymorphism.

### FIGURE LEGENDS

Figure 1. Human *FCN2* (GeneID 2220) genomic organization, shown for splice variants SV0 (NCBI reference sequence IDNM_004108.2) and SV1 (NCBI reference sequence ID NM_015837.2).
Figure 2. Human *FCN2* wild-type mRNA nucleic acid sequence (transcript variant SV0, NCBI Reference Sequence: NM_004108.2, SEQ ID NO: 1) and encoded amino acid sequence (one letter amino acid code, SEQ ID NO: 2). Nucleic acid sequence numbering is given above the sequence of the open reading frame and starts at the A of translation initiation codon ATG and ends at the G of translation termination codon TAG. Amino acid numbering is given below the sequence. The single nucleotide polymorphism SNP rs7851696 is indicated in bold and by the variant codon and corresponding serine.
Figure 3. Association between donor FCN2(Ala258Ser) SNP rs7851696 and graft survival. Survival of homozygous wild-type (GG) and SNP rs7851696 heterozygous (GT) kidney grafts was followed during 10 years.
Figure 4. FCN2 levels in serum of homozygous wild-type (GG) and SNP rs7851696 heterozygous (GT) patients and donors.
Figure 5. Expression of FCN2 mRNA and protein in pre-transplant tissue. A and B: qPCR kinetics and melting curve analysis. C: mRNA levels in homozygous wild-type (GG) and SNP rs7851696 heterozygous (GT) kidney tissue after correction for beta-actin mRNA levels. D: Inter-tubule protein expression of FCN2.
Figure 6. FCN2 binding to necrotic cells. A: dot plot analysis of Annexin-V binding versus 7-AAD in necrotic Jurkat cells. B: dot plot analysis of FCN2 binding versus 7-AAD in necrotic Jurkat cells. C: binding of FCN2 in homozygous wild-type (GG) and SNP rs7851696 heterozygous (GT) serum to necrotic Jurkat cells.

### DETAILED DESCRIPTION OF THE INVENTION

Unless indicated or defined otherwise, all terms used have their usual meaning in the art, which will be clear to the skilled person. Reference is further made to the standard handbooks, such as (Sambrook and Russell, 2001), as well as to the general background art cited herein.

It is an aim of the present invention to provide a method for prediction of renal transplantation outcome, for assessment of the risk of post-transplantation kidney rejection, and/or for selecting an appropriate treatment, therapeutic agent or therapeutic agent dosage regime for the treatment of post-transplantation kidney rejection.

The inventors found that the presence and/or absence of at least one nucleic acid polymorphism in the gene for FCN2 of the kidney allows for the prediction of renal transplantation outcome, for assessment of the risk of post-transplantation kidney rejection, and/or for selecting an appropriate treatment, therapeutic agent or therapeutic agent dosage regime for the treatment of post-transplantation kidney rejection.

As used herein, "prognosis" and "prediction" are synonyms. Prognosis or prediction may not be absolute, in that they may relate to statistically increased chances or risk, e.g. increased odds ratio or hazard ratio, of renal transplantation outcome.

Accordingly, the method of the present invention enables the skilled person to predict, or assists the skilled person in predicting, renal transplantation outcome. The method of the present invention further enables the skilled person to assess, or assists the skilled person in assessing, the risk of post-transplantation kidney rejection. The method of the present invention thus enables the skilled person to select, or assists the skilled person in selecting, an appropriate treatment of post-transplantation kidney rejection, or therapeutic agent or therapeutic agent dosage regime for the treatment of post-transplantation kidney rejection.

As used herein, renal transplantation outcome relates to the clinical outcome parameters after transplantation of the kidney. Clinical outcome parameters after kidney transplantation are known to those skilled in the art and comprise the occurrence of acute transplant rejection and the occurrence of anti-thymocyte globulin (ATG)-requiring (severe) rejection, delayed graft function (DGF, Israni et al., 2008), defined by the need for dialysis within the first week post-transplantation, estimated glomerular filtration rate (eGFR, Levey et al., 2006), and kidney allograft loss. Therefore, prediction of renal transplantation outcome refers to at least one of
- (risk of) post-translational kidney rejection, either acute or ATG-requiring,
- (risk of) DGF,
- (risk of) declined eGFR,
- (risk of) kidney allograft loss, and
- need or dosage of immunosuppressive therapy after transplantation.

In a first embodiment of the invention, the method comprises detecting the presence and/or absence of at least one nucleic acid polymorphism in the gene for ficolin-2 of the kidney.

As used herein, ficolin 2 (FCN2) refers to L-ficolin, a member of the ficolin protein family of oligomeric lectins, the 35 kDa monomers being composed of an amino-terminal cysteine-rich region, a collagen-like domain that consists of tandem repeats of Gly-Xaa-Yaa triplet sequences (where Xaa and Yaa represent any amino acid), a neck region, and a fibrinogen-like domain. In human serum, two types of ficolin, known as L-ficolin (P35, ficolin L, ficolin 2 or hucolin) and H-ficolin (Hakata antigen, ficolin 3 or thermolabile b2-macroglycoprotein), have been identified, and both of them have lectin activity. L-ficolin recognises GlcNAc and H-ficolin recognises GalNAc. Another ficolin known as M-ficolin (P35-related protein, Ficolin 1 or Ficolin A) is not considered to be a serum protein and is found in leucocytes and in the lungs. L-ficolin and H-ficolin activate the lectin-complement pathway in association with MASPs. Absolute ficolin deficiency states have not yet been described, although low levels in chemotherapy patients compared with healthy controls have been observed (Minchinton et al., 2004). Kilpatrick DC et al. (2003) did not find a significant relationship between plasma ficolin concentrations and chemotherapy-related infections. Atkinson et al. (2004) identified low L-ficolin concentrations in children with recurrent respiratory infections, although Ruskamp et al. (2009) found no correlation between single nucleotide polymorphisms in *FCN2* and respiratory tract infections in children. Recent studies showed a correlation of between single nucleotide polymorphisms in *FCN2,* together with single nucleotide polymorphisms in *MBL2* and *MASP2,* and the risk of bacterial and viral infections after orthotopic liver transplantation (de Rooij et al., 2010; de Rooij et al., 2011).

The term "nucleic acid" refers to a single-stranded or double-stranded polynucleotide which may consist of deoxyribonucleotides or ribonucleotides, nucleotide analogues or modified nucleotides, and may have been adapted. There is no limitation in length. The sequence of a nucleic acid or polynucleotide may be further referred to as nucleotide sequence.

The terms "protein", "polypeptide" and "peptide" all refer to a polymer of natural or non-natural amino acids, amino acid homologues, or modified amino acids, and may have been adapted. There is no limitation in length. The sequence of a protein or polypeptide may be further referred to as amino acid sequence.

The reference nucleotide sequences and amino acid sequences indicated in the current invention are derived from GeneBank (NCBI) and indicated by their respective accession number, as is well known to the person skilled in the art. The nomenclature for the ficolin nucleotide and amino acid changes as used herein is generally accepted and recommended by den Dunnen and Antonarakis (2000). Accordingly, the nucleotide numbering of the coding DNA and RNA reference sequence is as follows: nucleotide +1 is the A of the ATG-translation initiation codon, there is no nucleotide 0, the nucleotide 5' of the ATG-translation initiation codon is -1.

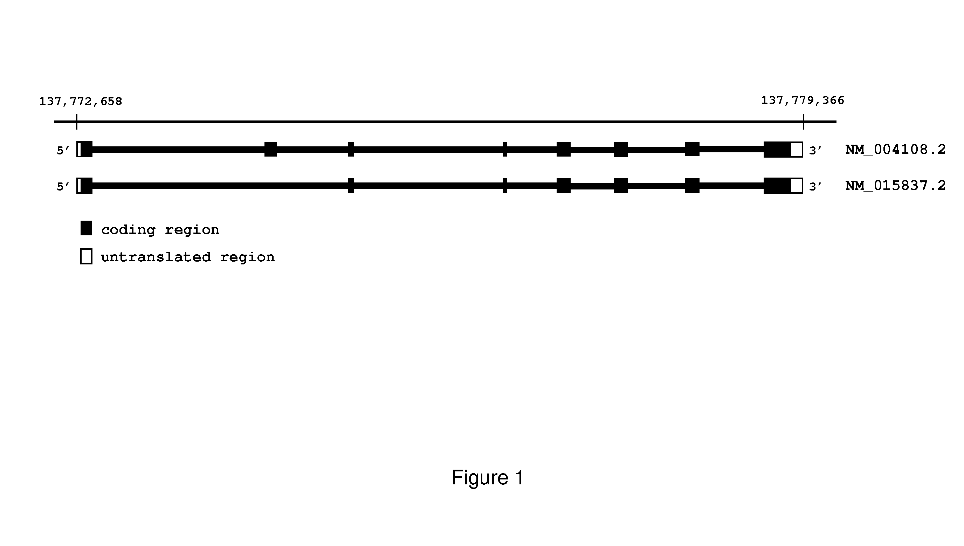

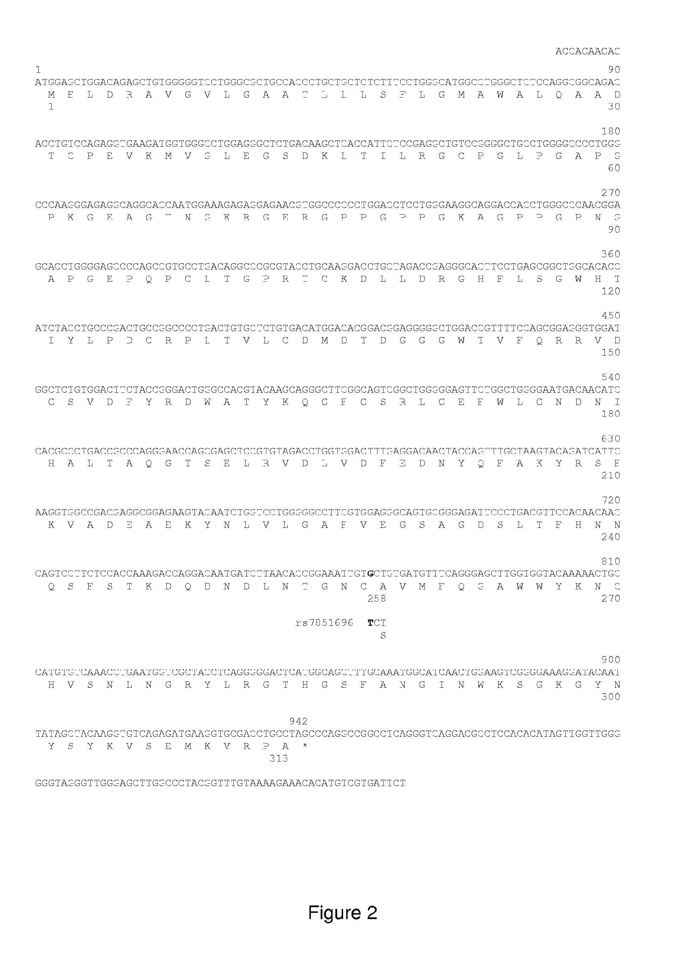

In humans, the ficolin-2 gene (*FCN2*, Entrez Gene ID 2220, NCBI Reference Sequence: NG_011649.1) is located together with *FCN1* on chromosome position 9q34. The genomic organization of *FCN2* is shown in Figure 1. Four different transcripts of the FCN2 gene arising from alternative splicing, and encoding different isoforms of ficolin 2, have been described. The splice variant SV0 (NCBI reference sequence ID NM_004108.2) is the most predominant FCN2 gene transcript in the liver and encodes a protein of 313 amino acids, called isoform a (NCBI reference sequence ID NP_004099.2). The SV0 splice variant mRNA is formed by splicing of eight exons after transcription of the genomic sequence. The mRNA reference sequence and translated amino acid sequence (one letter code) of human FCN2 SV0 are shown in Figure 2. The splice variant SV1 (NCBI reference sequence ID NM_015837.2) is a minor FCN2 gene transcript in the liver, and results from the skipping of exon 2 during splicing. Since the reading frame is unchanged by this splicing event, it encodes a shorter protein of 275 amino acids, called isoform b (NCBI reference sequence ID NP_056652.1). The splice variant SV2 (NCBI reference sequence ID NM_015838.1) is a minor FCN2 gene transcript in the liver, and is generated by the persistence of the fifth intron between exons 5 and 6. It encodes the same 143 amino acids in the amino-terminal end as the SV0 transcript variant, plus an additional 39 amino acids from the intron sequence (NCBI reference sequence ID NP_056653.1). The splice variant SV3 (NCBI reference sequence ID NM_015839.1) is a minor FCN2 gene transcript in the liver, and is generated by the persistence of introns 4 and 5. Exons 1-4 of this transcript encode a truncated protein of 102 amino acids, followed by an in-frame stop codon at the beginning of intron 4 (NCBI reference sequence ID NP_056654.1).

As used herein, "gene polymorphism", "genetic polymorphism", "nucleic acid polymorphism", "gene variation", "genetic variation", "nucleic acid variation", "gene variant", "genetic variant", and "nucleic acid variant" are used as synonyms, and all refer to a gene variant that differs in its nucleotide sequence from one or more reference nucleotide sequences, and to the actual difference or variation in nucleotide sequence between the genetic variant and a reference nucleotide sequence, as it is this difference or variation that defines the variant.

The most simple nucleic acid polymorphism is a polymorphism affecting a single nucleotide, i.e. a single nucleotide polymorphism (SNP). Nucleic acid polymorphisms further include any number of contiguous and/or non-contiguous differences in the nucleotide sequence of the nucleic acid under investigation relative to the nucleotide sequence of one or more reference nucleic acids. The term "polymorphic position" or "position" refers to the nucleic acid position at which a nucleic acid polymorphism arises or which characterizes the nucleic acid variant. A nucleic acid comprising at least one such polymorphism are referred to as "polymorphic nucleic acid", "polymorphic polynucleotide", or the like. The nucleotide sequence of a polymorphic nucleic acid is referred to as "variant nucleotide sequence" or "polymorphic nucleotide sequence". The polymorphism can be an insertion, deletion, substitution, tandem repeat, or similar.

As used herein, the term "allele" is one of several alternative forms of a gene or nucleic acid region at a specific chromosomal location (locus). For diploid organisms like humans, each autosomal locus of an individual possesses two alleles, one inherited from each parent. The allele which shows the lowest frequency in a particular population is called the "minor allele", while the allele which is most frequent in a particular population is called the "major allele". As used herein, "major allele", "reference allele", and "wild-type allele" are synonyms. Similarly, "minor allele", "polymorphic allele", and "variant allele" are synonyms. The nucleotide sequence of a wild-type allele of a locus is called the "wild-type nucleotide sequence" or "reference nucleotide sequence" of the locus.

The term "genotype" means the genetic constitution of an individual, either overall or at a specific locus.

A nucleic acid polymorphism is absent at a locus if the nucleotide sequence of the locus is identical to the reference nucleotide sequence or wild-type nucleotide sequence of that locus. A nucleic acid polymorphism is present at a locus if the nucleotide sequence of the locus differs from the reference nucleotide sequence or wild-type nucleotide sequence of that locus. For diploid organisms like humans, a nucleic acid polymorphism can be absent at a locus on both chromosomes, whereby the organism is called homozygous for the wild type or major allele. Similarly, a nucleic acid polymorphism can be present at a locus on both chromosomes, whereby the organism is called homozygous for the variant or minor allele. Also, a nucleic acid polymorphism can be absent at a locus on one chromosome and present at the same locus on the other chromosome, whereby the organism is called heterozygous for the major and minor alleles.

Thus in a first embodiment of the method of the present invention, the genotype of the kidney is investigated in relation to the presence and/or absence of at least one nucleic acid polymorphism in the gene for ficolin-2. It is clear to the skilled person that the genotype of the kidney is identical to that of the original kidney donor. Therefore, the investigation of the genotype of the kidney can be substituted by investigation of the genotype of the original kidney donor.

It will be apparent to the person skilled in the art that there are a large number of analytical procedures which may be used to detect the presence or absence of the nucleic acid polymorphisms mentioned herein. Nucleic acid from any nucleated cell can be used as the starting point for such assay techniques and may be isolated according to standard nucleic acid preparation procedures well known to those of skill in the art. Many current methods for the detection of allelic variation are reviewed by Nollau and Wagener (Nollau and Wagener, 1997), and in standard textbooks, for example "Laboratory Protocols for Mutation Detection", Ed. by U. Landegren, Oxford University Press, 1996 and "PCR, 2nd Edition" by Newton & Graham, BIOS Scientific Publishers Limited, 1997 (incorporated herein by reference).

The method of the present invention can be carried out *in vivo* or *in vitro.* Preferred, however, is *in vitro* detection of nucleic acid polymorphisms in the ficolin-2 gene in a biological sample obtained from the subject. The term "biological sample" means a tissue sample or a body fluid sample. A tissue sample includes, but is not limited to, buccal cells, a brain sample, a skin sample or organ sample, e.g. kidney. The term "body fluid" refers to all fluids that are present in the body including but not limited to blood, plasma, serum, lymph, urine, saliva or cerebrospinal fluid. The biological sample may also be obtained by subjecting it to a pretreatment if necessary, for example, by homogenizing or extracting. Such a pretreatment may be selected appropriately by those skilled in the art depending on the biological sample to be subjected.

A nucleic acid comprising an intended sequence prepared from a biological sample may be prepared from DNA (e.g. gDNA or cDNA) or RNA (e.g. mRNA). Release, concentration and isolation of the nucleic acids from the sample can be done by any method known in the art. Currently, various commercial kits are available. Other, well-known procedures for the isolation of DNA or RNA from a biological sample are also available (Sambrook and Russell, 2001).

When the quantity of the nucleic acid is low or insufficient for the assessment, the nucleic acid may be amplified. Such amplification procedures can be accomplished by those methods known in the art, including, for example, the polymerase chain reaction (PCR), ligase chain reaction (LCR), nucleic acid sequence-based amplification (NASBA), strand displacement amplification, rolling circle amplification, T7-polymerase amplification, and reverse transcription polymerase reaction (RT-PCR).

After performing the extraction and/or amplification procedure, the presence or absence of certain nucleic acid polymorphisms in the target sequence can be detected. Numerous methods for detecting nucleic acid polymorphisms are well-known in the art. The present invention is not limited by any particular method used to detect the target sequences disclosed herein. Examples of such methods are described by (Syvanen, 2001; Gut, 2001), and include, but are not limited to, hybridization methods such as reverse dot blot, Line Probe Assay (LiPA), bead arrays, geneChip microarrays, Dynamic Allele-Specific Hybridization (DASH), Peptide Nucleic Acid (PNA) and Locked Nucleic Acid (LNA) probes, TaqMan (5' nuclease assay) and molecular beacons; allele-specific PCR (ASP) methods such as Amplification Refractory Mutation System (ARMS), sequence-specific primers (SSP), Fluorescence Resonance Energy Transfer (FRET) primers and Alphascreen®; primer extension methods such as kinetic PCR, SNPstream®, Genetic Bit Analysis (GBA), multiplex minisequencing, SNaPshot, pyrosequencing, MassExtend, MassArray, GOOD assay, microarray minisequencing, Arrayed Primer EXtension (APEX), microarray primer extension, Tag arrays, coded microspheres, Template-Directed dye-Incorporation (TDI) assay, fluorescence polarization; oligonuceotide ligation methods such as colorimetric Oligonucleotide Ligation Assay (OLA), sequence-coded OLA, microarray ligation, ligase chain reaction, Multiplex Ligation-dependent Probe Amplification (MLPA), padlock probes and rolling circle amplification; endonuclease cleavage methods such as Restriction Fragment Length Polymorphism (RFLP) analysis and Invader® assay, and direct sequencing methods. In a preferred embodiment, the presence or absence of a nucleic acid polymorphism is determined by hybridization, sequencing, PCR, primer extension, MLPA, OLA or RFLP analysis.

In a preferred embodiment of the method of the invention, the nucleic acid polymorphism in *FCN2* of which the presence and/or absence is to be detected results in altered binding capacity of FCN2 to N-acetyl-D-glucosamine (GlcNAc).

Known ligands of FCN2 are GlcNAc, lipoteichoic acid, 1,3-β-D-glucan, and the capsular polysaccharide of type III group B streptococci (Garlatti et al., 2009). It has been determined in the present invention that nucleic acid polymorphisms in *FCN2* in the kidney genome that influence the binding capacity of FCN2 to its ligands influence renal transplantation outcome and the risk of post-transplantation kidney rejection. More in particular, it was found that nucleic acid polymorphisms in *FCN2* in the kidney genome that influence the binding capacity of FCN2 to GlcNAc influence renal transplantation outcome and the risk of post-transplantation kidney rejection. The FCN2(Ala258Ser) and FCN2(Thr236Met) variants show increased and decreased binding capacity to GlcNAc as compared to wild-type FCN2, respectively (Hummelshoj et al., 2005). More in particular, nucleic acid polymorphisms in *FCN2* in the kidney genome that result in increased binding capacity of FCN2 to GlcNAc result in improved renal transplantation outcome and decreased risk of post-transplantation kidney rejection.

Therefore, in a more preferred embodiment of the invention, the presence in the kidney genome of at least one nucleic acid polymorphism in *FCN2* resulting in increased binding capacity of FCN2 to GlcNAc is indicative of improved prognosis of renal transplantation outcome, of decreased risk of post-transplantation kidney rejection, and/or of decreased need of immunosuppression therapy in post-transplantational therapy.

In a preferred embodiment of the method of the invention, the nucleic acid polymorphism in *FCN2* in the kidney genome of which the presence and/or absence is to be detected leads to the substitution of alanine on position 258 of FCN2 by another amino acid.

In a more preferred embodiment of the invention, the nucleic acid polymorphism in *FCN2* in the kidney genome of which the presence and/or absence is to be detected leads to the substitution of alanine on position 258 of FCN2 by serine.

In a preferred embodiment of the method of the invention, the nucleic acid polymorphism is a single nucleotide polymorphism (SNP). To date, several SNPs have been identified in *FCN2* (see Table 1 for a non-limiting list of examples). A nucleic acid polymorphism to be detected in the method of the present invention may be any of these known SNPs or any other SNP, the presence and/or absence of which is indicative of improved prognosis of renal transplantation outcome, of decreased risk of post-transplantation kidney rejection, and/or of decreased need of immunosuppression therapy in post-transplantational therapy. Preferably, the SNP to be detected in the method of the present invention results in altered binding capacity of the protein encoded by the gene for ficolin-2 to N-acetyl-D-glucosamine (GlcNAc). More preferably, the SNP to be detected in the method of the present invention results in increased binding capacity of the protein encoded by the gene for ficolin-2 to N-acetyl-D-glucosamine (GlcNAc).

The FCN2(Ala258Ser) and FCN2(Thr236Met) variants, which show increased and decreased binding capacity to GlcNAc as compared to wild-type FCN2, respectively, are the consequence of the presence of SNP rs7851696 and SNP rs17549193, respectively, both located in exon 8. The first SNP is located at position 6424 of the genomic sequence, corresponding to position 772 of the mRNA or cDNA, whereby G is replaced by T. The latter SNP is located at position 6359 of the genomic sequence, corresponding to position 707 of the mRNA or cDNA, whereby C is replaced by T. In an even more preferable embodiment of method of the present invention, the nucleic acid polymorphism the presence and/or absence of which is detected is SNP rs7851696.

**Table 1. List of SNPs identified in and near the FCN2 gene. Nucleic acid changes listed are either substitutions (e.g. A>C) relative to the reference or wild-type sequence of FCN2 at the indicated position, or insertions (e.g. ->G) into the reference or wild-type sequence of FCN2 after the indicated position.**

| **SNP** | **Position on genome relative to ATG** | **Position on mRNA relative to ATG** | **Location** | **Nucleic acid change** | **Codon** | **Amino acid change** |
|---|---|---|---|---|---|---|
| rs78368215 | -1977 | - | 5' near gene | A>C | | |
| rs4350050 | -1950 | - | 5' near gene | C>T | | |
| rs74706412 | -1821 | - | 5' near gene | C>T | | |
| rs114754331 | -1764 | - | 5' near gene | A>T | | |
| rs3128628 | -1728 | - | 5' near gene | C>T | | |
| rs116314896 | -1524 | - | 5' near gene | G>T | | |
| rs77570339 | -1252 | - | 5' near gene | A>G | | |
| rs55848654 | -1248 | - | 5' near gene | A>C | | |
| rs55669323 | -1231 | - | 5' near gene | G>T | | |
| rs56014433 | -1011 | - | 5' near gene | A>G | | |
| rs55690104 | -1003 | - | 5' near gene | A>C | | |
| rs72551115 | -987 | - | 5' near gene | G>T | | |
| rs3124952 | -985 | - | 5' near gene | A>G | | |
| rs55726384 | -958 | - | 5' near gene | A>G | | |
| rs3811144 | -936 | - | 5' near gene | A>G | | |
| rs72551116 | -913 | - | 5' near gene | C>T | | |
| rs3811143 | -901 | - | 5' near gene | G>T | | |
| rs72551117 | -848 | - | 5' near gene | C>T | | |
| rs72551118 | -819 | - | 5' near gene | C>T | | |
| rs76739162 | -721 | - | 5' near gene | C>T | | |
| rs118040565 | -668 | - | 5' near gene | A>C | | |
| rs3124953 | -601 | - | 5' near gene | A>G | | |
| rs3811141 | -569 | - | 5' near gene | G>T | | |
| rs3811140 | -556 | - | 5' near gene | C>T | | |
| rs72551119 | -552 | - | 5' near gene | A>G | | |
| rs74693003 | -417 | - | 5' near gene | A>G | | |
| rs116872072 | -300 | - | 5' near gene | A>G | | |
| rs72551120 | -293 | - | 5' near gene | A>G | | |
| rs55809110 | -250 | - | 5' near gene | A>G | | |
| rs12344051 | -213 | - | 5' near gene | A>G | | |
| rs3811139 | -170 | - | 5' near gene | A>G | | |
| rs72551121 | -68 | - | 5' near gene | A>G | | |
| rs7865453 | -63 | - | 5' near gene | A>C | | |
| rs17514136 | -4 | -4 | 5' UTR | A>G | - | |
| rs61736807 | 11 | 11 | exon 1 | A>T | 4 | Asp>Val |
| rs72551122 | 12 | 12 | exon 1 | C>A | 4 | Asp>Glu |
| rs55797213 | 33 | 33 | exon 1 | C>T | 11 | Gly>Gly |
| rs3128627 | 125 | - | intron 1 | A>G | | |
| rs7032741 | 270 | - | intron 1 | C>T | | |
| rs74887236 | 328 | - | intron 1 | A>G | | |
| rs112246750 | 424 | - | intron 1 | C>G | | |
| rs72776336 | 682 | - | intron 1 | C>T | | |
| rs77021007 | 694 | - | intron 1 | C>T | | |
| rs114736855 | 806 | - | intron 1 | C>T | | |
| rs3124954 | 829 | - | intron 1 | C>T | | |
| rs73565971 | 868 | - | intron 1 | A>G | | |
| rs3128626 | 889 | - | intron 1 | C>T | | |
| rs116314839 | 922 | - | intron 1 | A>G | | |
| rs3128625 | 951 | - | intron 1 | A>G | | |
| rs78935511 | 1003 | - | intron 1 | C>T | | |
| rs72776338 | 1033 | - | intron 1 | A>G | | |
| rs79435897 | 1111 | - | intron 1 | A>G | | |
| rs11103562 | 1120 | - | intron 1 | A>T | | |
| rs74361720 | 1140 | - | intron 1 | C>T | | |
| rs74381095 | 1229 | - | intron 1 | G>T | | |
| rs77793455 | 1258 | - | intron 1 | A>G | | |
| rs114756253 | 1358 | - | intron 1 | A>G | | |
| rs4370608 | 1611 | - | intron 1 | A>G | | |
| rs4578023 | 1620 | - | intron 1 | A>G | | |
| rs55865317 | 1766 | 162 | exon 2 | G>A | 54 | Gly>Gly |
| rs80189097 | 1842 | - | intron 2 | C>G | | |
| rs3124955 | 1878 | - | intron 2 | C>T | | |
| rs4370609 | 1896 | - | intron 2 | A>G | | |
| rs75577478 | 1898 | - | intron 2 | C>G | | |
| rs4509412 | 1902 | - | intron 2 | A>C | | |
| rs4604523 | 1976 | - | intron 2 | A>G | | |
| rs73565972 | 1989 | - | intron 2 | C>T | | |
| rs113851583 | 1990 | - | intron 2 | A>G | | |
| rs77862660 | 2035 | - | intron 2 | C>T | | |
| rs73565973 | 2051 | - | intron 2 | C>T | | |
| rs73565979 | 2088 | - | intron 2 | C>T | | |
| rs111467869 | 2089 | - | intron 2 | A>G | | |
| rs12344423 | 2158 | - | intron 2 | C>T | | |
| rs3124956 | 2182 | - | intron 2 | A>G | | |
| rs12340783 | 2199 | - | intron 2 | C>G | | |
| rs115161307 | 2269 | - | intron 2 | C>T | | |
| rs116110178 | 2293 | - | intron 2 | A>G | | |
| rs12340835 | 2349 | - | intron 2 | A>G | | |
| rs73565985 | 2384 | - | intron 2 | A>T | | |
| rs7024491 | 2417 | - | intron 2 | A>G | | |
| rs118122273 | 2422 | - | intron 2 | A>G | | |
| rs3128624 | 2472 | - | intron 2 | C>T | | |
| rs4520243 | 2488 | 222 | exon 3 | T>C | 74 | Arg>Arg |
| rs113565542 | 2492 | 226 | exon 3 | C>T | 76 | Pro>Ser |
| rs111325668 | 2495 | 229 | exon 3 | C>G | 77 | Pro>Ala |
| rs7037264 | 2545 | - | intron 3 | A>G | | |
| rs79375627 | 2735 | - | intron 3 | C>T | | |
| rs75082791 | 2781 | - | intron 3 | ->G | | |
| rs67911189 | 2782 | - | intron 3 | ->G | | |
| rs74208368 | 2786 | - | intron 3 | ->G | | |
| rs12236429 | 2917 | - | intron 3 | A>G | | |
| rs117643848 | 3020 | - | intron 3 | G>T | | |
| rs117871363 | 3158 | - | intron 3 | G>T | | |
| rs111669839 | 3162 | - | intron 3 | G>T | | |
| rs11445130 | 3166 | - | intron 3 | ->G | | |
| rs67302903 | 3167 | - | intron 3 | ->G | | |
| rs7041446 | 3387 | - | intron 3 | A>G | | |
| rs77613398 | 3393 | - | intron 3 | A>G | | |
| rs3128623 | 3442 | - | intron 3 | C>T | | |
| rs11792008 | 3451 | - | intron 3 | A>C | | |
| rs7041633 | 3546 | - | intron 3 | C>G | | |
| rs71506944 | 3587 | - | intron 3 | A>G | | |
| rs7023162 | 3733 | - | intron 3 | C>T | | |
| rs75281646 | 3773 | - | intron 3 | A>G | | |
| rs74909701 | 3847 | - | intron 3 | A>G | | |
| rs115414303 | 3887 | - | intron 3 | A>T | | |
| rs55985459 | 3980 | - | intron 4 | C>T | | |
| rs12685659 | 4052 | - | intron 4 | A>T | | |
| rs71506945 | 4139 | - | intron 4 | C>T | | |
| rs12684459 | 4191 | - | intron 4 | A>G | | |
| rs116782100 | 4364 | - | intron 4 | C>T | | |
| rs79264803 | 4368 | - | intron 4 | C>T | | |
| rs111538932 | 4415 | - | intron 4 | C>T | | |
| rs55895215 | 4423 | 307 | exon 5 | C>T | 103 | Arg>Cys |
| rs17549179 | 4453 | 337 | exon 5 | C>T | 113 | His>Tyr |
| rs12684476 | 4465 | 349 | exon 5 | G>A | 117 | Gly>Ser |
| rs56084543 | 4526 | 410 | exon 5 | C>T | 137 | Thr>Met |
| rs55841373 | 4551 | - | intron 5 | C>T | | |
| rs56103000 | 4553 | - | intron 5 | A>T | | |
| rs76665625 | 4577 | - | intron 5 | C>G | | |
| rs55834586 | 4588 | - | intron 5 | A>G | | |
| rs75123259 | 4647 | - | intron 5 | C>T | | |
| rs113899750 | 4701 | - | intron 5 | C>T | | |
| rs56117058 | 4704 | - | intron 5 | C>G | | |
| rs77254375 | 4806 | - | intron 5 | A>G | | |
| rs12684512 | 4837 | - | intron 5 | A>G | | |
| rs117241205 | 4852 | - | intron 5 | C>T | | |
| rs56200327 | 4888 | - | intron 5 | C>T | | |
| rs56235352 | 4957 | 440 | exon 6 | G>A | 147 | Arg>Gln |
| rs78251566 | 4986 | 469 | exon 6 | C>T | 157 | Arg>Trp |
| rs56281005 | 4987 | 470 | exon 6 | G>A | 157 | Arg>Gln |
| rs34789496 | 5060 | 543 | exon 6 | C>T | 181 | His>His |
| rs111618014 | 5069 | 552 | exon 6 | C>T | 184 | Thr>Thr |
| rs55860122 | 5070 | 553 | exon 6 | G>A | 185 | Ala>Thr |
| rs12684723 | 5121 | - | intron 6 | A>G | | |
| rs113043031 | 5125 | - | intron 6 | C>T | | |
| rs115695257 | 5269 | - | intron 6 | A>G | | |
| rs76690858 | 5360 | - | intron 6 | G>T | | |
| rs76328175 | 5473 | - | intron 6 | A>G | | |
| rs80319672 | 5483 | - | intron 6 | C>T | | |
| rs79892543 | 5572 | - | intron 6 | A>G | | |
| rs73565996 | 5579 | - | intron 6 | C>T | | |
| rs113041810 | 5614 | 565 | exon 7 | A>G | 189 | Ser>Gly |
| rs11103563 | 6031 | - | intron 7 | A>G | | |
| rs62573178 | 6183 | - | intron 7 | A>G | | |
| rs10125317 | 6216 | - | intron 7 | C>T | | |
| rs7872508 | 6220 | - | intron 7 | G>T | | |
| rs113125899 | 6345 | - | intron 7 | A>G | | |
| rs17549193 | 6359 | 707 | exon 8 | C>T | 236 | Thr>Met |
| rs7851696 | 6424 | 772 | exon 8 | G>T | 258 | Ala>Ser |
| rs28357091 | 6443 | 791 | exon 8 | C>A | 264 | Ala>Asp |
| rs55874707 | 6459 | | | C>T | | |
| rs117435384 | 6536 | 884 | exon 8 | C>T | 295 | Ser>Leu |
| rs76267164 | 6584 | 932 | exon 8 | G>A | 311 | Arg>Gln |
| rs113204016 | 6639 | 987 | 3'UTR | ->T | - | |
| rs79955924 | 6647 | 995 | 3'UTR | A>T | - | |
| rs76316648 | 6648 | 996 | 3'UTR | A>G | - | |
| rs74554327 | 6850 | - | 3' near gene | A>C | | |
| rs73664188 | 6940 | - | 3' near gene | C>T | | |
| rs78020799 | 7049 | - | 3' near gene | C>T | | |
| rs12115600 | 7066 | - | 3' near gene | A>G | | |
| rs114831455 | 7087 | - | 3' near gene | C>T | | |
| rs7045822 | 7159 | - | 3' near gene | A>G | | |

The nucleic acid polymorphism, the presence and/or absence of which is to be detected in the method of the present invention, can be present in only one copy of *FCN2,* such that the kidney is heterozygous for the presence of the nucleic acid polymorphism, or it can be present in both copies of *FCN2,* whereby the kidney is homozygous for the presence of the nucleic acid polymorphism. Alternatively, the nucleic acid polymorphism may be absent in both copies of *FCN2,* whereby the kidney is homozygous for the wild-type allele.

In a preferred embodiment, the present invention relates to a method according to the present invention, wherein the nucleic acid polymorphism, the presence and/or absence of which is to be detected, is present in only one copy of *FCN2,* such that the kidney is heterozygous for the presence of the nucleic acid polymorphism.

In a more preferred embodiment of the method of the present invention, the kidney being heterozygous for the presence of said nucleic acid polymorphism is indicative of improved prognosis of renal transplantation outcome, of decreased risk of post-transplantation kidney rejection, and/or of decreased need of immunosuppression therapy in post-transplantational therapy, while the kidney being homozygous for the absence of said nucleic acid polymorphism is indicative of poorer prognosis of renal transplantation outcome, of increased risk of post-transplantation kidney rejection, and/or of increased need of immunosuppression therapy in post-transplantational therapy.

As used herein, improved prognosis of renal transplantation outcome relates to improved clinical outcome parameters after transplantation of the kidney. Clinical outcome parameters after kidney transplantation are known to those skilled in the art and comprise the occurrence of acute transplant rejection and the occurrence of anti-thymocyte globulin (ATG)-requiring (severe) rejection, delayed graft function (DGF, Israni et al., 2008), defined by the need for dialysis within the first week post-transplantation, estimated glomerular filtration rate (eGFR, Levey et al., 2006), and kidney allograft loss. Therefore, improved prognosis of renal transplantation outcome refers to at least one of
- decreased risk of post-translational kidney rejection, either acute or ATG-requiring,
- decreased risk of DGF,
- decreased risk of declined eGFR,
- decreased risk of kidney allograft loss, and
- decreased need of immunosuppressive therapy after transplantation.

In a preferred embodiment of the method of the invention, the presence and/or absence of said nucleic acid polymorphism is determined using at least one nucleic acid polymorphism-specific oligonucleotide. In a more preferred embodiment of the method of the invention, said nucleic acid polymorphism-specific oligonucleotide is a SNP rs7851696-specific oligonucleotide. In an even more preferred embodiment of the method of the present invention, the presence and/or absence of the nucleic acid polymorphism is determined using a further oligonucleotide which is specific for the wild-type allele corresponding to the polymorphism.

The polymorphism located in the *FCN2* gene may also be detected *in vitro* by determining in the FCN2 protein encoded by the *FCN2* gene, the presence and/or absence of an amino acid change resulting from the nucleic acid polymorphism by sequencing said protein. The amino acid change may also be detected by any conventional method known in the art, for example by mass-spectroscopy, gel electrophoresis, MALDI-TOF mass spectroscopy, FCN2 protein variant-specific ELISA, protein arrays, determination of the molecular weight, or by isoelectrofocusing.

Accordingly, the present invention provides a method for prediction of renal transplantation outcome, for assessment of the risk of post-transplantation kidney rejection, and/or for selecting an appropriate treatment, therapeutic agent or therapeutic agent dosage regime for the treatment of post-transplantation kidney rejection, comprising determining the presence and/or absence of at least one amino acid change in FCN2 of the kidney.

The polymorphism located in the *FCN2* gene may also be detected *in vitro* by determining the ligand-binding capacity, e.g. GlcNAc-binding capacity, of the FCN2 protein present in a sample of the kidney or original kidney donor, e.g. serum. Non-limiting examples of assays which can be used or adapted for such determination are taught in Hummelshoj et al (Hummelshoj et al., 2005), which is incorporated herein in its entirety. In a first assay, ELISA wells coated with GlcNAc-BSA are incubated with dilution series of the sample to be tested, and captured FCN2 is determined by anti-FCN2 antibody and if needed a detectable second antibody. An increased GlcNAc-binding capacity of the FCN2 protein will be reflected in an increased detection signal, independent of the actual sample concentration of FCN2. In a second assay, GlcNAc beads are incubated with the sample to be tested, and after washing captured FCN2 is eluted by addition of free GlcNAc. Eluted FCN2 is then determined using ELISA. Irrespective of the assay used, by comparing the GlcNAc-binding capacity in the tested sample to the GlcNAc-binding capacity in samples of known genotypes, the skilled person is able to conclude whether the GlcNAc-binding capacity in the tested sample is altered or not.

Accordingly, the present invention provides a method for prediction of renal transplantation outcome, for assessment of the risk of post-transplantation kidney rejection, and/or for selecting an appropriate treatment, therapeutic agent or therapeutic agent dosage regime for the treatment of post-transplantation kidney rejection, comprising determining the ligand-binding capacity in a sample of the kidney or original kidney donor. In a preferred embodiment of the method of the invention, the ligand-binding capacity is the GlcNAc-binding capacity.

In another embodiment of the present invention, oligonucleotides are provided which are specific for the nucleic acid polymorphism of which the presence and/or absence is to be detected. In a preferred embodiment, the oligonucleotides of the invention are primers or probes. In another preferred embodiment, the oligonucleotides of the invention are immobilized to a solid phase.

Polymorphism-specific and corresponding wild-type-specific oligonucleotides for use in the methods of the invention are of any convenient length and comprise nucleotide sequences, specifically hybridizing to the target nucleic acid, of at least 8, 9, 10, 11, 12, 13, 14 or 15 nucleotides and up to 40 nucleotides, up to 30 nucleotides or more conveniently up to 25 nucleotides, such as for example 8, 9, 10, 11, 12, 13, 14, 15, 16, 17, 18, 19, 20, 21, 22, 23, 24 or 25 nucleotides. In general such oligonucleotides will comprise nucleotide sequences entirely complementary to the polymorphism or corresponding wild type sequence in the *FCN2* gene. However, if required one or more nucleotides may be added or one or more mismatches may be introduced, provided that the discriminatory power of the oligonucleotides is not unduly affected. Specific length and sequence of the oligonucleotides will depend on the complexity of the required nucleic acid target, as well as on the reaction conditions such as temperature and ionic strength. In general, the hybridization conditions are to be stringent as known in the art. The primers or probes of the invention may carry one or more labels to facilitate detection.

In a preferred embodiment, the oligonucleotide consists of 10 to 60 nucleotides, preferably 15 to 30 nucleotides, and is capable of specifically forming a hybrid with a part of the *FCN2* gene and is at least one or more selected from the group consisting of:
- an oligonucleotide capable of hybridizing under stringent conditions with the sequence as represented by SEQ ID NO: 1, or the complementary thereof;
- an oligonucleotide of which the sequence is 80, 85 or 90% identical to the sequence as represented by SEQ ID NO: 1, or the complementary thereof; and
- an oligonucleotide capable of hybridizing under stringent conditions with the sequence as represented by SEQ ID NO: 1 wherein one or more nucleotides was subjected to a variation such as a substitution, deletion, insertion or addition, or the complementary thereof.

Further provided by the present invention are kits comprising at least one nucleic acid polymorphism-specific oligonucleotide, for use in a method for prediction of renal transplantation outcome, for assessment of the risk of post-transplantation kidney rejection, and/or for selecting an appropriate treatment, therapeutic agent or therapeutic agent dosage regime for the treatment of post-transplantation kidney rejection. In a preferred embodiment, the kits of the invention further comprise at least one oligonucleotide which is specific for the wild-type allele corresponding to said nucleic acid polymorphism. In another preferred embodiment of the kits of the invention, the oligonucleotides are immobilized to a solid phase.

### EXAMPLES

### Example 1: Investigation of innate immunity SNPs in relation to clinical outcome.

### Patients and donors

For DNA analysis, we selected all patients who had been transplanted between 1995 and 2005 with a kidney from a cadaveric, HLA-DR compatible donor in the Leiden University Medical Center (LUMC) in Leiden or in the Erasmus Medical Center (EMC) in Rotterdam. The entire study population consisted of 520 transplant recipients (50% LUMC, 50% EMC) and their donors.

### SNP analysis: PCR reactions and signal detection

With the in-house developed system 81 SNPs in 22 different innate immunity genes (Table 2) were investigated in both patient and donor DNA. The SNPs had initially been selected due to their localization in an exon, an intron (up to 30 bp before an exon; up to 10 bp after an exon) or a promoter region/5'-untranslated region (5' UTR) (up to 1,000 bp before exon 1).

**Table 2: SNPs investigated in the present study.**

| class | gene | SNP rs number | | class | gene | SNP rs number |
|---|---|---|---|---|---|---|
| MBL | MBL2 | rs11003125 | | Signaling | TLR1 | rs5743551 |
| | MBL2 | rs7096206 | | | TLR1 | rs5743611 |
| | MBL2 | rs7095891 | | | TLR1 | rs4833095 |
| | MBL2 | rs5030737 | | | TLR1 | rs3923647 |
| | MBL2 | rs1800450 | | | TLR2 | rs1898830 |
| | MBL2 | rs1800451 | | | TLR2 | rs3804100 |
| Secreted | Masp1-3 | rs710474 | | | TLR2 | rs5743708 |
| | Masp3 | rs850312 | | | TLR2 | +2029C>T (R677W)* |
| | Masp1 | rs3733001 | | | TLR3 | rs5743305 |
| | Masp2 | rs72550870 | | | TLR3 | rs3775296 |
| | Masp2 | rs2273343 | | | TLR3 | rs3775291 |
| | Masp2 | rs6695096 | | | TLR4 | rs1927914 |
| | Masp2 | rs223346 | | | TLR4 | rs4986790 |
| | Masp2 | rs12711521 | | | TLR4 | rs4986791 |
| | Masp2 | rs1782455 | | | TLR5 | rs759303 |
| | FCN1 | rs2989727 | | | TLR5 | rs5744168 |
| | FCN1 | rs1071583 | | | TLR5 | rs2072493 |
| | FCN2 | rs7865453 | | | TLR5 | rs5744174 |
| | FCN2 | rs17514136 | | | TLR6 | rs1039559 |
| | FCN2 | rs17549193 | | | TLR6 | rs5743810 |
| | FCN2 | rs7851696 | | | TLR6 | rs3821985 |
| | FCN3 | rs3813800 | | | TLR6 | rs5743815 |
| | C1qR1 | rs3746731 | | | TLR7 | rs5743712 |
| | C1qR1 | rs7492 | | | TLR7(*) | rs2897827 |
| Signaling | LBP | rs2232578 | | | TLR7 | rs179008 |
| | LBP | rs5744204 | | | TLR7 | rs5743781 |
| | LBP | rs2232607 | | | TLR8 | rs1548731 |
| | LBP | rs1780627 | | | TLR8 | rs3764880 |
| | LBP | rs2232613 | | | TLR8 | rs5744077 |
| | LBP | rs2232618 | | | TLR8 | rs2159377 |
| | CD14 | rs3138078 | | | TLR8 | rs5744080 |
| | CD14 | rs2569190 | | | TLR9 | rs187084 |
| | CD14 | rs2228049 | | | TLR9 | rs5743836 |
| | CD14 | rs2563298 | | | TLR9 | rs352140 |
| | LY96 | rs1809440 | | | TLR10 | rs7694115 |
| | LY96 | rs6472812 | | | TLR10 | rs11466645 |
| | LY96 | rs11466004 | | | TLR10 | rs11096957 |
| | Card15 | rs2066844 | | | TLR10 | rs11096956 |
| | Card15 | rs2066845 | | | TLR10 | rs4129009 |
| | Card15 | rs2066847 | | | | |
| | Sigirr | rs7482596 | | | | |
| | Sigirr | rs3210908 | | | | |
| | Sigirr | rs3087588 | | | | |

| | | | | | | |
|---|---|---|---|---|---|---|
| * no rs number assigned | | | | | | |

The following genes and number of SNPs (between brackets) were investigated: *TLR1* (4), *TLR2* (4), *TLR3 (3), TLR4* (3), *TLR5* (4), *TLR6* (4), *TLR7* (3), *TLR8* (5), *TLR9* (3), *TLR10* (5), *MASP1*/*MASP3* (3), *MASP2 (6), FCN1* (2), *FCN2* (4), *FCN3* (1), *C1QR1* (2), *LBP* (6), *CD14* (3), *LY96* (2), *SIGIRR* (3), *NOD2*/*CARD15* (3). Additionally, six DNA polymorphisms in the *MBL2* gene were investigated in DNA from patients only, using an MBL2 line probe assay. This assay includes analysis of three mutations in the promoter and 5' UTR of the *MBL2* gene, affecting MBL expression, and three mutations in exon 1 that affect functionality of the MBL2 protein. For analysis, A/A A/O and O/O variants were established with the use of the three SNPs in exon 1 (Berger et al., 2007).

DNA (~125 ng) was amplified by PCR: five multiplex PCRs (*MBL2*; *MASP1* + *MASP2* + *MASP3* + *FCN1* + *FCN2 + FCN3 + C1QR1*; *LBP* + *CD14* + *LY96* + *SIGIRR*; *TLR1* to -5; *TLR6* to *-10*) and one single PCR *(CARD15)* were carried out. In the PCR reactions 3.3 mM MgCl₂ and 1.5-3.5 U of HotStart Taq enzyme was added (1.5 U for *MBL2* and *NOD2*/*CARD15*; 3.5 U for the other PCR reactions). The amplified DNA was denatured, and was allowed to hybridize to probes, which had been coated as parallel lines on a nitrocellulose membrane.

The probes have been designed to hybridize specifically with their complementary sequences. Hybridization, wash steps, and visualization of the DNA products were performed in Auto-LiPA devices (INNOGENETICS, Zwijnaarde, Belgium). Hybridized products were visualized on the basis of conversion of a chromogen (NBT/BCIP) to a purple precipitate by biotin/streptavidin/alkaline phosphatase complexes. For each SNP, the dots on the membranes were interpreted using software, and they reflected either the wild type, heterozygous or mutant variant. Approximately, 481 DNA samples from patients and 473 DNA samples from donors could be reliably interpreted for genotype analysis.

### Clinical outcome parameters and statistics

Genotypes with a frequency of less than 5% in the total population were left out of the analyses. SNPs were associated with the following two clinical outcome parameters: occurrence of acute rejection (yes/no) after transplantation and occurrence of ATG-requiring (severe) rejection (yes/no). In multiple SNP testing strategies we wanted to limit the possibility of finding prognostic gene markers by chance. Therefore, the patient cohort was divided randomly into a definition set (~1/3 of the total group) and a validation set (the remaining 2/3 of the group). The clinical relevance of any of the SNPs tested was considered significant only when an association (chi-square test) with the outcome parameter(s) with a P<0.05 was found in both the definition set and the validation set. SNPs showing statistically significant associations were further analyzed in the following way:
1) they were included in a multivariate model together with clinical risk factors to test for independent prediction of the clinical outcome parameters.
2) they were tested for an association with graft loss (censored for death with functioning graft) using Kaplan-Meier curves and log-rank testing.
3) they were included together with demographic and clinical parameters in a multivariate Cox regression model.

### Example 2: Association of donor FCN2 genotype with DGF.

A second patient cohort was employed to study association between FCN2 genotype and occurrence of delayed graft function (DGF, defined as dialysis-dependency within the first week post-transplant). DNA was available for 144 patients and 130 donors. Forward and reverse sequencing primers targeting exon 8 of *FCN2* have been described previously (Hummelshoj et al., 2005). The primers were extended with M13 tails (5'-GAC-GTT-GTA-AAA-CGA-CGG-CCA-GT-3'). PCR reactions were purified with ExoSAP-IT® (Isogen Life Science). Sequence reactions were purified according to the protocol from the BigDye® Terminator (v1.1) kit (Applied Biosystems). Sequencing reactions were performed with the 3730xl DNA Analyzer (Applied Biosystems).

### Example 3: Investigation of FCN2 expression levels.

### Serum

FCN2 levels were measured in serum from a subset of patients and donors included in the patient cohort, using a human FCN2 ELISA kit (Cell Sciences, Canton MA, USA). Serum was investigated from 36 patients homozygous for the variant of FCN2 (GG), from 39 patients heterozygous for SNP rs7851696 (GT), and from 3 patients homozygous for SNP rs7851696 (TT). Sera had been taken 43 ± 36 days before transplantation. Furthermore, serum was investigated from 21 GG donors and 11 GT donors (donor sera were kindly provided by Dr. Eric Claas, Dept. of Medical Microbiology, Leiden).

### Pre-transplant material: mRNA and protein expression

We investigated whether mRNA expression is present in pre-transplant kidneys. Sixty-nine wedge biopsies before transplantation (57 GG, 12 GT), not related to the original patient cohort, were available for study. RNA extraction and qPCR was performed according to previously described protocols (19,20), and using FCN2-specific primer sequences CGA-GGC-GGA-GAA-GTA-CAA-TC (forward) and TGG-CAG-TTT-TTG-TAC-CAC-CA (reverse). Liver cDNA was used as positive control to optimize the PCR reaction. Beta-actin was used as reference gene to correct for cDNA input. For localization of FCN2 protein in the kidney, immunohistochemistry was carried out with anti-FCN2 antibody on tissue sections from four different cadaveric donor kidneys, which had been disapproved for transplantation purposes.

### Example 4: Binding of FCN2 to dying cells

For cell death experiments Jurkat T cells were used (0.5 x 10⁶ per culture). After harvesting cells were spun (7 min, 400xg) and resuspended 1:20 in IMDM/1 containing 10% FCS and penicillin/streptomycin. To obtain apoptotic cells, Jurkat T cells were irradiated with UV at 100 Joule, and were analyzed either 4 h later (early apoptotic) or 24 h later (late apoptotic). For necrosis, cells were incubated for 30 min in a 56°C water bath. Cells were washed with HEPES buffer (5 mM CaCl₂, 155 mM NaCl, 25 mM HEPES, pH 7.4). FCN2 was then added for 1h at 37°C, either in recombinant form (positive control) or as part of total serum. In the latter case, sera were pre-incubated for 20 min with 1.31 mg/mL blocking antibody against C1q. Cells were washed with HEPES buffer containing 5% heat-inactivated FCS, incubated for 30 min at 4°C with FCN2 antibodies (Abcam, ab56225; 1:100 diluted), washed again, and then incubated for 15 min at 4°C with 5 µg/mL goat-anti-mouse IgG-PE (Dako, R0480). Cell death markers were 7-AAD (BD, 51-68981E) and Annexin-V-APC (B&D). The number of cells positive for Annexin-V, 7-AAD, and FCN2 were determined with flow cytometry.

To detect differences in binding capacity to dying cells between FCN2 genotype variants, six sera were diluted in such a way that the FCN2 content was 3 µg/mL. From this point the sera were further diluted in a two-fold dilution series, and incubated with 0.5 x 10⁶ necrotic Jurkat T cells. Three sera had the wild type FCN2 variant (GG), and three sera contained FCN2 with the Ala258Ser variant. Only cases which were homozygous for the wild-type variant at position 707 in exon 8 of the mRNA (i.e. having Thr at position 236 of FCN2) were selected.

### Example 5: Investigation of innate immunity SNPs in relation to transplant outcome.

Single nucleotide polymorphisms (SNPs) in innate immunity markers were studied in relation to incidence of rejection and to incidence of ATG-requiring rejection. In the definition set 11 SNPs were significantly (P<0.05) associated with rejection incidence (Table 3). In the validation set, two SNPs in the *TLR9* gene in the donors were associated with rejection incidence: one was located in the promoter region and the other in exon 1. Since for both these SNPs the genotype distribution differed between the definition set and the validation set (data not shown), they were not further investigated in graft survival plots.

Eight SNPs in the definition set were significantly associated with incidence of ATG-requiring rejection (Table 3). Two SNPs additionally showed significance in the validation set: a SNP in the 3'-untranslated region of the *TLR3* gene in the patients and an amino-acid substituting SNP in exon 8 of the *FCN2* gene (Ala258Ser) in the donors. The *TLR3* SNP did not show an impact on graft survival (data not shown). The *FCN2* SNP (rs7851696) was further investigated.

**Table 3. Associations between SNPs and rejection incidence ¹**

| **Rejection** (yes/no) | | | |
|---|---|---|---|
| | | Definition set | Validation set ² |
| Patient | TLR3 IVS1 | 0.025 | 0.075 |
| | TLR8 ex3b | 0.037 | 0.424 |
| | TLR9 prom1a a | 0.028 | 0.259 |
| | CD14 ex2b | 0.037 | 0.942 |
| | MASP2IVS8 | 0.034 | 0.147 |
| Donor | TLR1 ex4b | 0.033 | 0.614 |
| | TLR5 ex6b | 0.039 | 0.306 |
| | TLR9 prom1a a | 0.046 | 0.030* |
| | TLR9 ex2 | 0.024 | 0.023 * |
| | LBP ex10a | 0.024 | 0.326 |
| | C1qR1 ex2 | 0.006 | 0.234 |
| | | | |

| **ATG-requiring rejection** (yes/no) | | | |
|---|---|---|---|
| | | Definition set | Validation set ² |
| Patient | TLR3 IVS1 | 0.037 | 0.018* |
| | CD14 ex2b | 0.020 | 0.821 |
| Donor | LBP prom | 0.021 | 0.818 |
| | LBP ex10a | 0.013 | 0.228 |
| | LBP ex13 | 0.007 | 0.950 |
| | FCN1 ex9 | 0.024 | 0.998 |
| | FCN2 ex8b | 0.042 | 0.028* |
| | C1qR1 ex2 | 0.001 | 0.083 |

| | | | |
|---|---|---|---|
| ¹ ex, exon; IVS, intervening sequence ; prom, promoter. ² * denotes significant P value in the validation set | | | |

### Example 6: Donor FCN2(Ala258Ser) SNP rs7851696 and transplant outcome

In the definition set the GG kidneys (homozygous wild type, frequency: 77.2%) and GT kidneys (heterozygous for SNP rs7851696, frequency: 21.8%) showed an incidence of 22.6% and 8.9%, respectively, of ATG-requiring rejection in the patients (Table 4, P=0.042). In the validation set these incidences were approximately the same, namely 21.2% and 8.6%, respectively (Table 4, P=0.028). The upstream Thr236Met SNP rs17549193 (frequencies: homozygous wilt-type, CC: 48.8%, heterozygous variant, CT: 44.0%, homozygous variant, TT: 7.2%) was not significantly associated to the outcome parameters (data not shown).

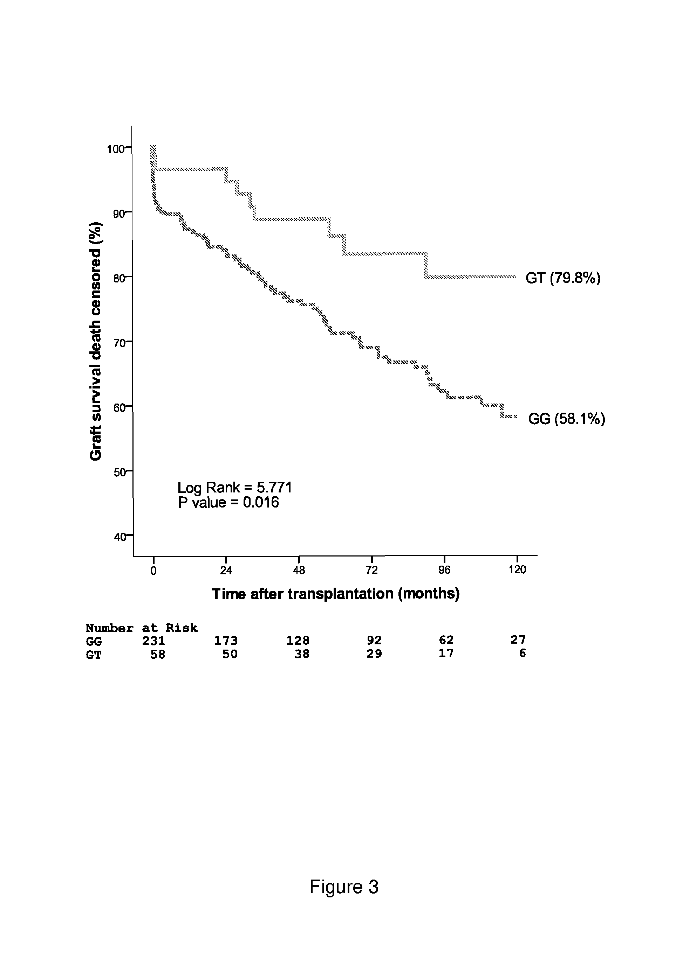

It was further found that patients, who had received a transplant from a GT donor, had significantly better graft survival (Figure 3, 79.8% after 10 years) than patients who had received a kidney transplant from a GG donor (58.1% after 10 years, P=0.016).

**Table 4. Association between donor FCN2(Ala258Ser) SNP rs7851696 and lower incidence of ATG-requiring rejection.**

| **Definition set (n=182)** | | | | |
|---|---|---|---|---|
| P=0.042 | | ATG-requiring rejection | | Total |
| | | No | Yes | |
| Donor *FCN2* exon 8 genotype | GG | 106 | 31 | 137 |
| | | 77.4% | 22.6% | 100.0% |
| | GT | 41 | 4 | 45 |
| | | 91.1% | 8.9% | 100.0% |
| Total | | 147 | 35 | 182 |
| | | 80.8% | 19.2% | 100.0% |
| | | | | |

| **Validation set (n=289)** | | | | |
|---|---|---|---|---|
| P=0.028 | | ATG-requiring rejection | | Total |
| | | No | Yes | |
| Donor *FCN2* exon 8 genotype | GG | 182 | 49 | 231 |
| | | 78.8% | 21.2% | 100.0% |
| | GT | 53 | 5 | 58 |
| | | 91.4% | 8.6% | 100.0% |
| Total | | 235 | 54 | 289 |
| | | 81.3% | 18.7% | 100.0% |

Next, it was investigated whether donor SNP rs7851696 is an independent predictor of graft outcome. This SNP was tested in univariate and multivariate regression analysis together with clinical and demographic parameters that included period of transplantation, current and historic panel reactive antibodies, cold ischemia time, medical center, number of transplants received so far, patient and donor gender, patient and donor age, and HLA class I mismatching. A GG donor kidney resulted in a three times higher risk for severe rejection as compared to a GT donor kidney, independently of clinical variables (OR=3.13, P=0.026) (Table 5). In multivariate Cox regression analysis (Table 4) the GG donor kidney showed a significant hazard ratio of 2.04 (P=0.046).

**Table 5. Univariate and multivariate analysis for graft outcome.**

| **Logistic regression (occurrence severe rejection) ¹** | | | | |
|---|---|---|---|---|
| | Univariate | | Multivariate | |
| | OR (95% CI) | P value | OR (95% CI) | P value |
| Donor FCN2 variant | 2.85 (1.08-7.53) | 0.034 | 3.13 (1.15-8.54) | 0.026 |
| Patient age | 0.46 (0.25-0.83) | 0.010 | 0.43 (0.22-0.85) | 0.014 |
| Period of transplantation | 0.35 (0.19-0.65) | 0.001 | 0.28 (0.14-0.57) | <0.001 |
| | | | | |

| **Cox regression (graft survival) ²** | | | | |
|---|---|---|---|---|
| | Univariate | | Multivariate | |
| | HR (95% CI) | P value | HR (95% CI) | P value |
| Donor FCN2 variant | 2.28 (1.14-4.56) | 0.020 | 2.04 (1.01-4.10) | 0.046 |
| Donor age | 2.31 (1.51-3.54) | <0.001 | 2.60 (1.64-4.11) | <0.001 |
| Center | 1.88 (1.22-2.89) | 0.004 | 1.81 (1.14-2.87) | 0.012 |
| HLA class I mismatching | 1.67 (1.04-2.70) | 0.036 | 1.84 (1.10-3.09) | 0.020 |
| Number of transplants | 1.62 (0.98-2.67) | 0.061 | 1.99 (1.18-3.39) | 0.011 |
| Period of transplantation | 0.65 (0.41-1.05) | 0.077 | 0.59 (0.35-0.98) | 0.043 |

| | | | | |
|---|---|---|---|---|
| ¹ OR, odds ratio; CI, confidence interval ² HR, hazard ratio | | | | |

In summary, the *FCN2* genotype of the kidney (donor), independently influences the outcome of kidney transplantation.

### Example 7: Association between donor FCN2 genotype and early transplant function

Within the first 3 months after transplantation almost 10% of the grafts in the group with FCN2 GG donors was already lost (see also graft survival plot in Figure 2). After censoring for patient death with functioning graft, thrombosis and/or infarction (43%) and permanent non-function of the graft (29%) accounted for most of the losses. In contrast, in the group with FCN2 GT grafts only one graft was lost within the first 3 months, and this was caused by hyperacute rejection.

The course after transplantation was then divided into two time frames: up to 2 years post-transplant and beyond 2 years. Indeed, the strongest divergence in graft survival between GG and GT grafts was observed in the first two years (84% and 96%, respectively, P=0.016, data not shown). With the graft survival set to 100% at the two-year time point, the graft survival plots for GG (69%) and GT grafts (83%) further diverged beyond two years (data not shown), but this difference was not significant, probably due to the lower number of cases.

In a second, independent cohort of kidney transplant recipients (n=144) and donors (n=130) we found that the donor SNP rs7851696 was protective against delayed graft function (DGF) (GG: 38.7%, GT: 17.4%, P=0.052). This association was not found for the SNP present in the patients *FCN2* (GG: 29.5%, GT: 28.2%), nor in the upstream Thr236Met SNP rs17549193 in donors (CC: 32.8%, CT: 35.8%, TT: 46.2%) and in patients (CC: 28.2%, CT: 30.0%, TT: 33.3%).

### Example 8: Investigation of FCN2 expression levels

### Serum levels

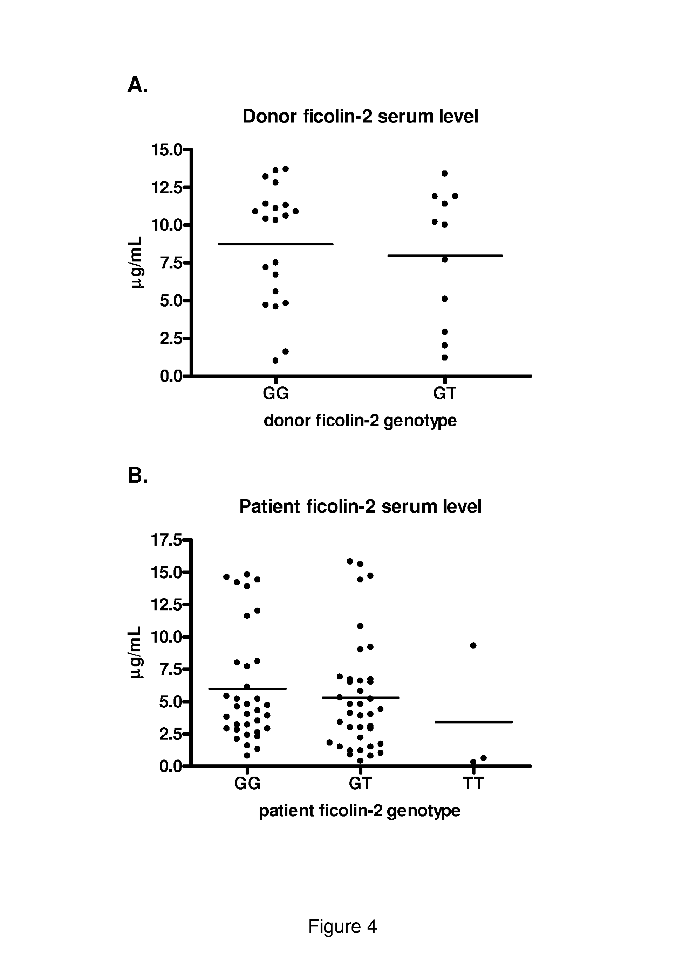

FCN2 levels in serum of GG patients (6.0 ± 4.4 µg/mL) were not significantly different from those in GT patients (5.3 ± 4.2 µg/mL) or TT patients (3.4 ± 5.1 µg/mL) (Figure 4A). Likewise, investigation of FCN2 levels in serum from donors did not show differences between GG donors (8.8 ± 3.9 µg/mL) and GT donors (8.0 ± 4.4 µg/mL) (Figure 4B).

### Pre-transplant intragraft expression

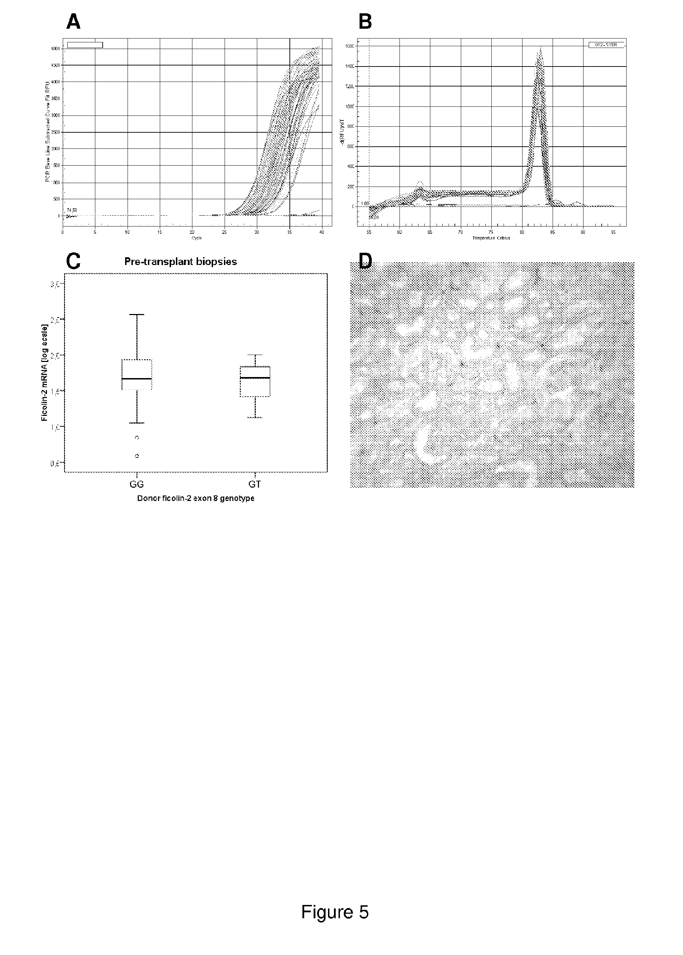

Messenger RNA expression for FCN2 was detected in all 69 pre-transplant biopsies studied: the average Ct value was 28.3 ± 1.6 (Figure 5A), indicating detectable transcription within the kidneys. In the melting curve analysis a single sharp peek was seen (Figure 5B), indicating one single, specific PCR product for FCN2. mRNA levels of FCN2 in the biopsies, after correction for beta-actin message, were not affected by genotype (Figure 5C).

Protein expression was detected in donor kidneys between the tubules (Figure 5D). The pattern seemingly resembles that of passenger leukocytes. We also found that monocyte-derived macrophages (cultured for a maximum of 7 days with M-CSF or GM-CSF) express significant amounts of FCN2 mRNA (data not shown).

### Example 9: Binding of FCN2 variants to its ligands

### Binding to GlcNAc

FCN2 primarily binds *N*-acetyl glucosamine (GlcNAc) residues on pathogens and can mediate the lectin pathway of complement activation. The *FCN2* SNP rs7851696, leads to a functional modification in the fibrinogen-like domain of the protein, and results in higher binding capacity for GlcNAc.

### Binding to dying cells

Since it has been shown that the Ala258Ser variant of FCN2 has significantly larger binding capacity to GlcNAc than the wild-type FCN2 (Hummelshoj et al., 2005), binding to dying cells by wild-type and variant FCN2 was investigated.

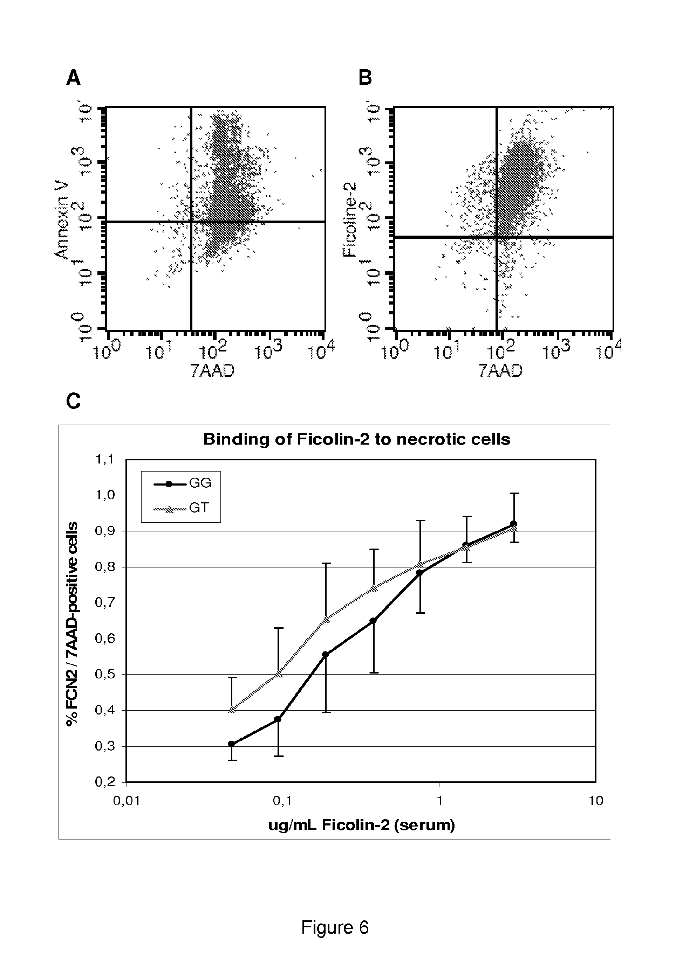

Necrotic cells were homogeneous with respect to expression of cell death markers. Virtually all cells were positive for both Annexin-V and 7-AAD (Figure 6A). Incubation with ≥3 µg/mL of FCN2 resulted into more than 90% of 7-AAD-positive cells being bound by FCN2 (Figure 6B). The number of cells bound by FCN2 in sera containing the Ala258Ser variant of FCN2 was slightly higher compared to sera containing only the wild type FCN2 (Figure 6C). This difference became prominent with higher dilutions of the sera, i.e. with <0.75 µg/mL of FCN2.

### REFERENCES

Alegre, M. L., Leemans, J., Le Moine, A., Florquin, S., De, Wilde, V, Chong, A., and Goldman, M. The multiple facets of toll-like receptors in transplantation biology. Transplantation. 86[1], 1-9. 15-7-2008.
Atkinson, A. P., Cedzynski, M., Szemraj, J., St Swierzko, A., Bak-Romaniszyn, L., Banasik, M., Zeman, K., Matsushita, M., Turner, M. L., and Kilpatrick, D. C. L-ficolin in children with recurrent respiratory infections. Clin Exp Immunol. 138[3], 517-520. 2004.
Berger, S. P., Roos, A., Mallat, M. J., Fujita, T., de Fijter, J. W., and Daha, M. R. Association between mannose-binding lectin levels and graft survival in kidney transplantation. Am J Transplant. 5[6], 1361-1366. 2005.
Berger, S. P., Roos, A., Mallat, M. J., Schaapherder, A. F., Doxiadis, I. I., van Kooten, C., Dekker, F. W., Daha, M. R., and de Fijter, J. W. Low pretransplantation mannose-binding lectin levels predict superior patient and graft survival after simultaneous pancreas-kidney transplantation. J Am Soc Nephrol. 18[8], 2416-2422. 2007.
Bouwman, L. H., Roep, B. O., and Roos, A. Mannose-binding lectin: clinical implications for infection, transplantation, and autoimmunity. Hum Immunol. 67[4-5], 247-256. 2006.
de Rooij, B. J., van der Beek, M. T., van Hoek, B., Vossen, A. C., Hove, W. R., Roos, A., Schaapherder, A. F., Porte, R. J., van der Reijden, J. J., Coenraad, M. J., Hommes, D. W., and Verspaget, H. W. Mannose-binding lectin and ficolin-2 gene polymorphisms predispose to cytomegalovirus (re)infection after orthotopic liver transplantation. J Hepatol. 17-2-2011.
de Rooij, B. J., van Hoek, B., ten Hove, W. R., Roos, A., Bouwman, L. H., Schaapherder, A. F., Porte, R. J., Daha, M. R., van der Reijden, J. J., Coenraad, M. J., Ringers, J., Baranski, A. G., Hepkema, B. G., Hommes, D. W., and Verspaget, H. W. Lectin complement pathway gene profile of donor and recipient determine the risk of bacterial infections after orthotopic liver transplantation. Hepatology. 52[3], 1100-1110. 2010.
den Dunnen, J. T. and Antonarakis, S. E. Mutation nomenclature extensions and suggestions to describe complex mutations: a discussion. Hum Mutat. 15[1], 7-12. 2000.
Ducloux, D., Deschamps, M., Yannaraki, M., Ferrand, C., Bamoulid, J., Saas, P., Kazory, A., Chalopin, J. M., and Tiberghien, P. Relevance of Toll-like receptor-4 polymorphisms in renal transplantation. Kidney Int. 67[6], 2454-2461. 2005.
Garlatti, V., Martin, L., Lacroix, M., Gout, E., Arlaud, G. J., Thielens, N. M., and Gaboriaud, C. Structural insights into the recognition properties of human ficolins. J Innate.Immun. 2[1], 17-23. 2009.
Goldstein, D. R. Toll like receptors and acute allograft rejection. Transpl.Immunol. 17[1], 11-15. 2006.
Goldstein, D. R. The identity of innate immune activators in organ transplantation: origins from within or exterior to the host? Am J Transplant. 7[7], 1692-1694. 2007.
Gut, I. G. Automation in genotyping of single nucleotide polymorphisms. Hum Mutat. 17[6], 475-492. 2001.
Hummelshoj, T., Munthe-Fog, L., Madsen, H. O., Fujita, T., Matsushita, M., and Garred, P. Polymorphisms in the FCN2 gene determine serum variation and function of Ficolin-2. Hum Mol Genet 14[12], 1651-1658. 15-6-2005.
Israni, A. K., Li, N., Cizman, B. B., Snyder, J., Abrams, J., Joffe, M., Rebbeck, T., and Feldman, H. I. Association of donor inflammation- and apoptosis-related genotypes and delayed allograft function after kidney transplantation. Am J Kidney Dis. 52[2], 331-339. 2008.
Jensen, M. L., Honore, C., Hummelshoj, T., Hansen, B. E., Madsen, H. O., and Garred, P. Ficolin-2 recognizes DNA and participates in the clearance of dying host cells. Mol Immunol. 44[5], 856-865. 2007.
Kilpatrick, D. C., McLintock, L. A., Allan, E. K., Copland, M., Fujita, T., Jordanides, N. E., Koch, C., Matsushita, M., Shiraki, H., Stewart, K., Tsujimura, M., Turner, M. L., Franklin, I. M., and Holyoake, T. L. No strong relationship between mannan binding lectin or plasma ficolins and chemotherapy-related infections. Clin Exp.Immunol. 134[2], 279-284. 2003.
Kruger, B., Krick, S., Dhillon, N., Lerner, S. M., Ames, S., Bromberg, J. S., Lin, M., Walsh, L., Vella, J., Fischereder, M., Kramer, B. K., Colvin, R. B., Heeger, P. S., Murphy, B. T., and Schroppel, B. Donor Toll-like receptor 4 contributes to ischemia and reperfusion injury following human kidney transplantation. Proc Natl Acad Sci U S.A. 106[9], 3390-3395. 3-3-2009.
Kuraya, M., Ming, Z., Liu, X., Matsushita, M., and Fujita, T. Specific binding of L-ficolin and H-ficolin to apoptotic cells leads to complement activation. Immunobiology. 209[9], 689-697. 2005.
Leemans, J. C., Stokman, G., Claessen, N., Rouschop, K. M., Teske, G. J., Kirschning, C. J., Akira, S., van der, Poll T., Weening, J. J., and Florquin, S. Renal-associated TLR2 mediates ischemia/reperfusion injury in the kidney. J Clin Invest. 115[10], 2894-2903. 2005.
Levey, A. S., Coresh, J., Greene, T., Stevens, L. A., Zhang, Y. L., Hendriksen, S., Kusek, J. W., and Van Lente, F. Using standardized serum creatinine values in the modification of diet in renal disease study equation for estimating glomerular filtration rate. Ann Intern Med. 145[4], 247-254. 15-8-2006.
Minchinton, R. M., Liley, H., and Eisen, D. P. The body as fortress: innate immune surveillance. Vox.Sang. 87 Suppl1, 30-34. 2004.
Nauta, A. J., Raaschou-Jensen, N., Roos, A., Daha, M. R., Madsen, H. 0., Borrias-Essers, M. C., Ryder, L. P., Koch, C., and Garred, P. Mannose-binding lectin engagement with late apoptotic and necrotic cells. Eur.J Immunol. 33[10], 2853-2863. 2003.
Nollau, P. and Wagener, C. Methods for detection of point mutations: performance and quality assessment. The IFCC Scientific Division, Committee on Molecular Biology Techniques. J Int Fed.Clin Chem. 9[4], 162-170. 1997.
Obhrai, J. and Goldstein, D. R. The role of toll-like receptors in solid organ transplantation. Transplantation. 81[4], 497-502. 27-2-2006.
Ogden, C. A., deCathelineau, A., Hoffmann, P. R., Bratton, D., Ghebrehiwet, B., Fadok, V. A., and Henson, P. M. C1q and mannose binding lectin engagement of cell surface calreticulin and CD91 initiates macropinocytosis and uptake of apoptotic cells. J Exp Med. 194[6], 781-795. 17-9-2001.
Palmer, S. M., Burch, L. H., Davis, R. D., Herczyk, W. F., Howell, D. N., Reinsmoen, N. L., and Schwartz, D. A. The role of innate immunity in acute allograft rejection after lung transplantation. Am J Respir.Crit.Care Med. 168[6], 628-632. 15-9-2003.
Palmer, S. M., Burch, L. H., Mir, S., Smith, S. R., Kuo, P. C., Herczyk, W. F., Reinsmoen, N. L., and Schwartz, D. A. Donor polymorphisms in Toll-like receptor-4 influence the development of rejection after renal transplantation. Clin Transplant. 20[1], 30-36. 2006.
Ruskamp, J. M., Hoekstra, M. O., Postma, D. S., Kerkhof, M., Bottema, R. W., Koppelman, G. H., Rovers, M. M., Wijga, A. H., de Jongste, J. C., Brunekreef, B., and Sanders, E. A. Exploring the role of polymorphisms in ficolin genes in respiratory tract infections in children. Clin Exp Immunol. 155[3], 433-440.2009.
Sambrook,J. and Russell,D.W. (2001). Molecular cloning: A Laboratory Manual., F.Ausubel, ed. (Cold Spring Harbor, NY: Cold Spring Harbor Laboratory Press).
Scaffidi, P., Misteli, T., and Bianchi, M. E. Release of chromatin protein HMGB1 by necrotic cells triggers inflammation. Nature. 418[6894], 191-195. 11-7-2002.
Syvanen, A. C. Accessing genetic variation: genotyping single nucleotide polymorphisms. Nat Rev Genet. 2[12], 930-942. 2001.

## Claims

1. A method for prediction of renal transplantation outcome, for assessment of the risk of post-transplantation kidney rejection, and/or for selecting an appropriate treatment, therapeutic agent or therapeutic agent dosage regime for the treatment of post-transplantation kidney rejection, comprising detecting the presence and/or absence of at least one nucleic acid polymorphism in the gene for ficolin-2 of the kidney.

2. The method of claim 1, wherein said nucleic acid polymorphism results in altered binding capacity of the protein encoded by the gene for ficolin-2 to N-acetyl-D-glucosamine (GlcNAc).

3. The method of claim 1 or 2, wherein said nucleic acid polymorphism leads to the substitution of alanine on position 258 of the protein encoded by the gene for ficolin-2 by another amino acid.

4. The method of claim 3, wherein said another amino acid is serine.

5. The method of any of claims 1 to 4, wherein said nucleic acid polymorphism is a single nucleotide polymorphism (SNP).

6. The method of any of claims 1 to 5, wherein said nucleic acid polymorphism is SNP rs7851696.

7. The method of any of claims 1 to 6, whereby
- the kidney being heterozygous for the presence of said nucleic acid polymorphism is indicative of improved prognosis of renal transplantation outcome, of decreased risk of post-transplantation kidney rejection, and/or of decreased need of immunosuppression therapy after transplantation, and
- the kidney being homozygous for the absence of said nucleic acid polymorphism is indicative of poorer prognosis of renal transplantation outcome, of increased risk of post-transplantation kidney rejection, and/or of increased need of immunosuppression therapy after transplantation.

8. The method of any of claims 1 to 7 which is an in vitro method.

9. The method of any of claims 1 to 8, wherein the presence and/or absence of said nucleic acid polymorphism is determined using at least one nucleic acid polymorphism-specific oligonucleotide, preferably a SNP rs7851696-specific oligonucleotide.

10. An oligonucleotide which is specific for the nucleic acid polymorphism from any of claims 1 to 9, for use in a method for prediction of renal transplantation outcome, for assessment of the risk of post-transplantation kidney rejection, and/or for selecting an appropriate treatment, therapeutic agent or therapeutic agent dosage regime for the treatment of post-transplantation kidney rejection.

11. The oligonucleotide of claim 10, which is a primer or a probe.

12. The oligonucleotide of claim 10 or 11, which is immobilized to a solid phase.

13. A kit comprising at least one nucleic acid polymorphism-specific oligonucleotide according to any of claims 10 to 12, for use in a method for prediction of renal transplantation outcome, for assessment of the risk of post-transplantation kidney rejection, and/or for selecting an appropriate treatment, therapeutic agent or therapeutic agent dosage regime for the treatment of post-transplantation kidney rejection.

14. The kit of claim 13, further comprising at least one oligonucleotide which is specific for the wild-type allele corresponding to said nucleic acid polymorphism.

15. The kit of claim 14, wherein said oligonucleotides are immobilized to a solid phase.
